# EUROPÄISCHE PATENTANMELDUNG

(11) **EP 2 863 209 A1**
(43) Veröffentlichungstag der Anmeldung: **22.04.2015**
(21) Anmeldenummer: 14188426.2
(22) Anmeldetag: 10.10.2014
(51) Int. Cl.: G01N 21/64, A61B 5/00, A61B 1/06

(54) **Endoskopische, exoskopische oder mikroskopische Vorrichtung zur Fluoreszenzdiagnose**

(30) Priorität: 15.10.2013 DE 102013111368
(71) Anmelder: Karl Storz GmbH & Co. KG, 78532 Tuttlingen (DE)
(72) Erfinder: Ehrhardt, André, 78573 Wurmlingen (DE); Beck, Gerd, 78573 Wurmlingen (DE); Glöggler, Bernhard, 78532 Tuttlingen (DE); Irion, Klaus-Martin, 78576 Emmingen-Liptingen (DE); Hinding, Thomas, 78554 Aldingen (DE); Martin, Uwe, 78549 Spaichingen (DE)
(74) Vertreter: Witte, Weller & Partner Patentanwälte mbB

(57) **Zusammenfassung**

Eine endoskopische, exoskopische oder mikroskopische Vorrichtung zur Fluoreszenzdiagnose weist eine Lichtquelle (12) auf, die dazu ausgelegt ist, in einem Fluoreszenzmodus Licht in einem ersten Spektralbereich und Licht in einem zweiten Spektralbereich zu emittieren, wobei der zweite Spektralbereich von dem ersten Spektralbereich zumindest teilweise getrennt ist. Die Lichtquelle (12) weist zumindest ein erstes Halbleiterleuchtmittel (32) auf, das im Fluoreszenzmodus das Licht in dem ersten Spektralbereich emittiert. Die Lichtquelle (12) weist zumindest ein zweites Halbleiterleuchtmittel (34) aufweist, das in dem Fluoreszenzmodus das Licht in dem zweiten Spektralbereich emittiert. Die Vorrichtung weist eine Steuer- oder Regeleinrichtung (56) auf, die ein voreingestelltes Verhältnis aus einer ersten Intensität des Lichts im ersten Spektralbereich und einer zweiten Intensität des Lichtes im zweiten Spektralbereich konstant hält.

## Beschreibung

Die Erfindung betrifft eine endoskopische, exoskopische oder mikroskopische Vorrichtung zur Fluoreszenzdiagnose, mit einer Lichtquelle, die dazu ausgelegt ist, in einem Fluoreszenzmodus Licht in einem ersten Spektralbereich und Licht in einem zweiten Spektralbereich zu emittieren, wobei der zweite Spektralbereich von dem ersten Spektralbereich zumindest teilweise getrennt ist, wobei die Lichtquelle zumindest ein erstes Halbleiterleuchtmittel aufweist, das im Fluoreszenzmodus Licht in dem ersten Spektralbereich emittiert.

Eine solche Vorrichtung ist aus DE 10 2008 018 637 A1 bekannt.

Eine erfindungsgemäße Vorrichtung zur Fluoreszenzdiagnose der eingangs genannten Art wird vorzugsweise zu medizinischen Diagnosezwecken verwendet, kann aber auch zu technischen Diagnosezwecken in industriellen oder wissenschaftlichen Anwendungen verwendet werden.

Im Rahmen der medizinischen Fluoreszenzdiagnose wird eine Vorrichtung der eingangs genannten Art zur Beurteilung des Zustands von biologischem Gewebe, beispielsweise allgemein zur Gewebedifferenzierung, insbesondere zur Tumorerkennung, aber auch zur Erkennung der Durchblutung und Vitalität verwendet. Mit einer Vorrichtung zur Fluoreszenzdiagnose der eingangs genannten Art kann die Fluoreszenzdiagnose insbesondere *in vivo* durchgeführt werden.

Im medizinischen Bereich hat sich die Fluoreszenzdiagnose zu einer vielversprechenden Alternative bzw. Ergänzung in der Erkennung und Behandlung neoplastischer Veränderungen entwickelt. Die Fluoreszenzdiagnose beruht auf der Wechselwirkung von Licht einer geeigneten Wellenlänge mit einer im zu untersuchenden Gewebeareal vorhandenen fluoreszierenden Substanz. Dabei kann eine fluoreszierende Substanz ein Fluoreszenzfarbstoff sein, der zuvor in das zu untersuchende Gewebeareal eingebracht wurde, beispielsweise durch Verabreichen der fluoreszierenden Substanz selbst oder einer Vorstufe derselben an den zu untersuchenden Patienten. Eine fluoreszierende Substanz kann jedoch auch eine bereits im Zielgebiet vorhandene Substanz sein, beispielsweise eine gewebespezifische Substanz, die durch Licht in einem geeigneten Spektralbereich zur Autofluoreszenz angeregt wird. Die vorliegende Erfindung kann beide Fälle umfassen. Für die nachfolgenden Erläuterungen wird von dem Fall ausgegangen, dass die fluoreszierende Substanz ein Fluoreszenzfarbstoff ist, der exogen (von außen) in das zu untersuchende Gewebeareal eingebracht wird.

Bei den tumorselektiven Substanzen wird derzeit der 5-Aminolävulinsäure(5-ALA) bzw. ihrem Hexylester (5-ALAHE, Handelsname Hexvix®) eine entscheidende Rolle beigemessen. Sie ist eine körpereigene Substanz und Ausgangsprodukt der intrazellulären Hämbiosynthese. Nach mehreren Reaktionsschritten werden intrazellulär endogene Porphyrine, in erster Linie das Protoporphyrin IX (PPIX), synthetisiert. Letzteres ist das entscheidende Fluorochrom, das während der fluoreszenzdiagnostischen Untersuchung nachgewiesen wird. Dies gelingt allerdings nur, wenn 5-ALA in ausreichender Konzentration den befallenen Organen exogen zugeführt wird.

In derzeitigen Fluoreszenzdiagnosevorrichtungen wird als Lichtquelle eine Xenonlampe verwendet, die über ein flexibles Lichtübertragungssystem Fluoreszenzanregungslicht in ein Endoskop einkoppelt. Zur videotechnischen Dokumentation wird ein für die Fluoreszenzdiagnose angepasstes Kamerasystem eingesetzt.

Im Falle der herkömmlichen Fluoreszenzdiagnosesysteme, die eine Xenonlampen-basierte Lichtquelle aufweisen, werden im Beleuchtungsstrahlengang ein Beleuchtungsfilter und im Beobachtungsstrahlengang ein Beobachtungsfilter eingesetzt. Die spektralen Transmissionseigenschaften des Beleuchtungsfilters und des Beobachtungsfilters bestimmen zusammen die Intensität des zurückgestreuten kurzwelligen, im Falle von PPIX blauen Anregungslichtes, das der Beobachter wahrnimmt. Für einen optimalen Kontrast zwischen fluoreszierendem und nicht fluoreszierendem Gewebe darf das zur Detektion gelangende remittierte Blaulicht nicht zu intensiv sein, da es sonst die Fluoreszenz, im Fall von PPIX Rotfluoreszenz, zu stark überlagert. Andererseits ist eine gewisse Mindestbeimischung von remittiertem Blaulicht, die sog. Blauzunge, wünschenswert, um die Diagnostik zu verbessern. Es ist bekannt, dass zurückgestreutes Licht und Fluoreszenzlicht ungefähr vergleichbare Intensität aufweisen sollten, um einen guten Farbkontrast zu erhalten.

Ohne Beschränkung der Allgemeinheit ist im vorstehenden Sinne der erste Spektralbereich der Hauptspektralbereich der Anregung der Fluoreszenz, und der zweite Spektralbereich ist ein Nebenspektralbereich (beispielsweise die Blauzunge), wobei Licht in dem Nebenspektralbereich dem Fluoreszenzbild beigemischt wird.

Einen Nachteil einer Xenonlampen-basierten Lichtquelle stellt die relativ kurze Lebensdauer von Xenonlampen dar. Hersteller von Fluoreszenzdiagnosesystemen empfehlen daher, die Xenonlampe bzw. das Lampenmodul nach einer gewissen, relativ niedrigen Betriebsstundenzahl auszutauschen. Der Nachteil solcher herkömmlicher Fluoreszenzdiagnosesysteme besteht daher zum einen in dem durch den Austausch der Xenonlampe bedingten Kostenmehraufwand und die Stillstandszeiten, die durch die Wartung bzw. den Austausch bedingt sind.

Demgegenüber besitzen Halbleiterleuchtmittel wie Leuchtdioden, worunter in der vorliegenden Anmeldung sowohl anorganische als auch organische Halbleiterleuchtmittel zu verstehen sind, wie Leuchtdioden, Laserdioden, Superlumineszenzdioden und dgl., eine deutlich höhere Lebensdauer, wodurch die vorstehend genannten Nachteile vermieden werden können. Halbleiterleuchtmittel weisen heutzutage auch eine ausreichende Strahlungsleistung von mehreren Watt auf.

In dem eingangs genannten Dokument DE 10 2008 018 637 A1 wird vorgeschlagen, für die Lichtquelle zur Fluoreszenzanregung eine Laserdiode, Leuchtdiode oder Superlumineszenzdiode zu verwenden.

Aus DE 102 52 313 A1 ist eine Fluoreszenzdiagnosevorrichtung bekannt, die eine Anregungslichtquellenanordnung zur Emission von Licht in einem Spektralbereich der Anregung eines Fluoreszenzfarbstoffes und eine weitere Lichtquellenanordnung zur Emission von Licht in einem weiteren Spektralbereich aufweist, der das Fluoreszenzspektrum und das Anregungsspektrum nicht umfasst. Dabei ist eine Intensität der weiteren Lichtquellenordnung relativ zu einer Intensität der Anregungslichtquellenanordnung änderbar. Die Änderung der Intensität der weiteren Lichtquellenanordnung wird dort durch Einbringen unterschiedlicher Beleuchtungsfilter in den Beleuchtungsstrahlengang, durch Beleuchtungsfilter mit einstellbarer Transmission und dgl. realisiert. In einem Rechner ist ferner eine optimale Relation zwischen den Maximalintensitäten in den beiden Spektralbereichen gespeichert, die vorab so ermittelt wurde, dass ein Benutzer fluoreszierende Bereiche und angrenzende nicht fluoreszierende Bereiche im Fluoreszenzbild gleichzeitig mit gutem Kontrast wahrnehmen kann, ohne dass die Fluoreszenzstrahlung überstrahlt wird. Der Rechner steuert einen Motor zur Drehung eines Filterrades derart an, dass die Relation der Maximalintensitäten in den beiden Spektralbereichen im Wesentlichen der optimalen Relation entspricht. Bei dieser Vorrichtung stimmt der Benutzer die optimale Relation der Maximalintensität der weiteren Lichtquelle selbst ab, die dann in dem Rechner hinterlegt wird. Die Abstimmung des optimalen Intensitätsverhältnisses durch den Benutzer birgt jedoch die Gefahr falsch positiver und vor allem falsch negativer Diagnosestellung.

Als Lichtquelle, die sowohl die Anregungslichtquelle als auch die weitere Lichtquelle bildet, wird dort wie herkömmliich eine breitbandige Lichtquelle, beispielsweise eine Halogenlampe, verwendet, mit den zuvor beschriebenen Nachteilen.

Während Halbleiterleuchtmittel im Vergleich zu Xenonlampen-basierten Lichtquellen eine hohe Lebensdauer besitzen, variieren die optischen Eigenschaften von Halbleiterleuchtmitteln, z.B. die Zentralwellenlänge, die Halbwertsbreite und die Strahlungsleistung durch den Herstellungsprozess innerhalb bestimmter Grenzen. Darüber hinaus werden die optischen Eigenschaften sowie die Lebensdauer von Halbleiterleuchtmitteln durch Betriebsparameter, wie beispielsweise Stromstärke, Spannung und Temperatur, beeinflusst. Die optischen Eigenschaften können sich während des Betriebes (in der Regel) reversibel oder aber im Laufe der Lebensdauer (in der Regel) irreversibel ändern. Dies kann bei einem Einsatz derartiger Lichtquellen dazu führen, dass sich der Farbkontrast während einer Sitzung oder im Laufe der Zeit unerwünscht ändert, wodurch eine sichere Diagnose nicht gewährleistet ist.

Der Erfindung liegt die Aufgabe zugrunde, eine Vorrichtung der eingangs genannten Art dahingehend weiterzubilden, dass der für eine sichere Diagnose erforderliche Farbkontrast im Fluoreszenzbild gleich bleibt, und dies während einer Diagnosesitzung, als auch über die Lebensdauer der Lichtquelle.

Erfindungsgemäß wird diese Aufgabe hinsichtlich der eingangs genannten endoskopischen, exoskopischen oder mikroskopischen Vorrichtung zur Fluoreszenzdiagnose dadurch gelöst, dass die Lichtquelle zumindest ein zweites Halbleiterleuchtmittel aufweist, das in dem Fluoreszenzmodus das Licht in dem zweiten Spektralbereich emittiert, und durch eine Steuer- oder Regeleinrichtung, die ein voreingestelltes Verhältnis aus einer ersten Intensität des Lichtes im ersten Spektralbereich und einer zweiten Intensität des Lichtes im zweiten Spektralbereich konstant hält.

Die erfindungsgemäße Vorrichtung zur Fluoreszenzdiagnose weist somit zur Emission von Licht im Anregungsspektralbereich als auch zur Emission von Licht im Nebenspektralbereich (beispielsweise für die Blauzunge) Halbleiterleuchtmittel auf. Hierdurch wird zunächst der Nachteil einer vergleichsweise kurzen Lebensdauer herkömmlicher Lichtquellen von Fluoreszenzdiagnosevorrichtungen, die Xenon- oder Halogenlampen-basierte Lichtquellen aufweisen, behoben. Das technische Problem des Driftens der optischen Eigenschaften von Halbleiterleuchtmitteln wird durch eine Steuer- oder Regeleinrichtung behoben, die ein voreingestelltes Verhältnis der Lichtintensitäten im ersten Spektralbereich und im zweiten Spektralbereich konstant hält. Das voreingestellte Verhältnis der vorstehend genannten Lichtintensitäten ist dabei nicht benutzerbestimmt, sondern vorzugsweise zuvor klinisch ermittelt und in der Fluoreszenzdiagnosevorrichtung, insbesondere in der Steuer- oder Regeleinrichtung derselben, hinterlegt. Die Steuer- oder Regeleinrichtung der erfindungsgemäßen Fluoreszenzdiagnosevorrichtung sorgt nun dafür, dass das voreingestellte Intensitätsverhältnis sowohl im Rahmen einer Diagnosesitzung als auch über die Lebensdauer der Lichtquelle, d.h. die Lebensdauer der Halbleiterleuchtmittel, beibehalten wird, d.h. auch unabhängig von Betriebsparametern wie Stromstärke, Spannung und Temperatur und unabhängig von der Alterung der Halbleiterleuchtmittel. Die erfindungsgemäße Fluoreszenzdiagnosevorrichtung gewährleistet somit stets einen für eine sichere Diagnose erforderlichen gleichbleibenden Farbkontrast im Fluoreszenzbild.

Die Steuer- oder Regeleinrichtung kann als Steuereinrichtung oder als Regeleinrichtung ausgebildet sein. Die Steuer- oder Regeleinrichtung kann so ausgebildet sein, dass sie die Beaufschlagung zumindest eines der Halbleiterleuchtmittel mit Strom und/oder Spannung steuert oder regelt. Sie kann auch eine Steuerung oder Regelung der Temperatur zumindest eines der Halbleiterleuchtmittel mittels einer Kühlung/Heizung bewirken, um das voreingestellte Intensitätsverhältnis konstant zu halten.

Die Steuer- oder Regeleinrichtung kann insbesondere dazu ausgelegt sein, das voreingestellte Verhältnis aus der ersten und der zweiten Intensität auf Änderungen hin zu überwachen und bei detektierten Änderungen das Ist-Verhältnis auf das voreingestellte Verhältnis zurückzusetzen. Letzteres kann dadurch bewerkstelligt werden, dass die Steuer- oder Regeleinrichtung die Stromstärke und/oder Spannung, mit der bzw. denen zumindest eines der Halbleiterleuchtmittel betrieben wird, steuert oder regelt.

Der Vorteil einer Überwachung des voreingestellten Intensitätsverhältnisses besteht vor allem darin, dass individuelle Eigenschaften der einzelnen Halbleiterleuchtmittel, die von Halbleiterleuchtmittel zu Halbleiterleuchtmittel unterschiedlich sein können, stets bei der Steuerung oder Regelung berücksichtigt werden können, insbesondere individuelle Unterschiede der Halbleiterleuchtmittel, die sich erst oder vor allem im Laufe der Alterung der Halbleiterleuchtmittel ergeben.

In einer bevorzugten Ausgestaltung der vorstehend genannten Maßnahme weist die Steuer- oder Regeleinrichtung eine Messeinrichtung zum Messen der Intensitäten des von dem ersten Halbleiterleuchtmittel und dem zumindest einen zweiten Halbleiterleuchtmittel emittierten Lichts auf.

Diese Ausgestaltung einer Überwachung des voreingestellten Intensitätsverhältnisses ist vorteilhafterweise einfach zu realisieren, insbesondere kann als Messeinrichtung eine Photodiode verwendet werden, die auf Helligkeitsänderungen mit einer Änderung der Strom-Spannungs-Kennlinie der Photodiode reagiert.

Die erfindungsgemäße Vorrichtung ist, wie bei herkömmlichen Fluoreszenzdiagnosevorrichtungen, vorzugsweise auch mit einer Bilderfassungseinrichtung zur Erfassung eines Fluoreszenzbildes ausgestattet. Hierbei ist es bevorzugt, wenn die Steuer- oder Regeleinrichtung Bildsignale der Bilderfassungseinrichtung auswertet, um das voreingestellte Verhältnis auf Änderungen zu überwachen.

In dieser Ausgestaltung ist die Basis der Konstanthaltung des voreingestellten Intensitätsverhältnisses vorteilhafterweise das Fluoreszenzbild bzw. dessen Kontrast selbst, wobei Änderungen des voreingestellten Intensitätsverhältnisses, die sich in einer Änderung des Farbkontrastes im Fluoreszenzbild äußern, von der Bilderfassungseinrichtung erfasst und von der Steuer- oder Regeleinrichtung instantan zur Nachführung des Intensitäts-Ist-Verhältnisses auf das voreingestellte Intensitätsverhältnis verwendet werden.

Alternativ zu einer Überwachung des Ist-Verhältnisses auf Abweichungen zu dem voreingestellten Intensitätsverhältnis kann in der Steuer- oder Regeleinrichtung ein typischer zeitlicher Verlauf von Änderungen der Intensitäten des von dem ersten Halbleiterleuchtmittel und dem zumindest einen zweiten Halbleiterleuchtmittel emittierten Lichts gespeichert sein, wobei die Steuer- oder Regeleinrichtung anhand des typischen zeitlichen Verlaufes der Änderungen Korrekturwerte berechnet, um das voreingestellte Verhältnis konstant zu halten.

Diese Maßnahme beruht darauf, dass ein gleichbleibender Farbkontrast aufeinander abgestimmte Stromstärken des Halbleiterleuchtmittels, das das Anregungslicht emittiert, und des Halbleiterleuchtmittels, das den Nebenspektralbereich emittiert, voraussetzt. Die hierzu erforderlichen Stromstärken der Halbleiterleuchtmittel können experimentell im Labor und/oder in einer klinischen Evaluierung ermittelt werden. Diese ermittelten Werte werden anschließend in der Fluoreszenzdiagnosevorrichtung, beispielsweise in einem Speicherbaustein, der in der Anregungslichtquelle hinterlegt sein kann, abgespeichert. In dieser Ausgestaltung der Vorrichtung ist die Steuer- oder Regeleinrichtung zumindest in der Lage, ein auf Erfahrungswerten beruhendes Driften des voreingestellten Intensitätsverhältnisses auszugleichen, wobei das Konstanthalten des voreingestellten Intensitätsverhältnisses möglicherweise einer Toleranz unterliegt. In diesem Fall findet lediglich eine Steuerung der Stromstärke zumindest eines der Halbleiterleuchtmittel statt, jedoch keine Regelung. Nichtsdestoweniger hat diese Ausgestaltung den Vorteil eines einfacheren Aufbaus und einer geringeren Komplexität, weil keine Überwachung des Ist-Intensitätsverhältnisses erforderlich ist.

Vorzugsweise ist die Steuer- oder Regeleinrichtung in die Lichtquelle integriert.

Der Vorteil hierbei ist eine kompakte Bauweise der Fluoreszenzdiagnosevorrichtung.

Das zumindest eine erste Halbleiterleuchtmittel und/oder das zumindest eine zweite Halbleiterleuchtmittel ist bzw. sind eine Leuchtdiode (anorganisch (LED) oder organisch (OLED)), worunter auch eine Superlumineszenzdiode zu verstehen ist, eine Laserdiode, ein Leuchtdiodenarray oder ein Laserdiodenarray.

Vorzugsweise weist die Lichtquelle zumindest ein, vorzugsweise mehrere weitere Halbleiterleuchtmittel auf, die in einem Weißlichtmodus Weißlicht erzeugen.

So kann die Lichtquelle zusätzlich eine weiße Leuchtdiode oder ein Leuchtdiodenarray mit roten, grünen und blauen LEDs aufweisen, die in Kombination miteinander Weißlicht erzeugen.

Die erfindungsgemäße Vorrichtung kann vorzugsweise zwischen dem Fluoreszenzmodus und dem Weißlichtmodus umgeschaltet werden, wobei der Weißlichtmodus zur Orientierung des Benutzers im beobachteten Areal dient. Im Zusammenhang mit den vorstehend genannten Maßnahmen ist es bevorzugt, wenn das zweite Halbleiterleuchtmittel eine einzelne Leuchtdiode ist, die im Fluoreszenzmodus nur zusammen mit dem ersten Halbleiterleuchtmittel aktiv ist, während das zweite Halbleiterleuchtmittel im Weißlichtmodus mit den weiteren Halbleiterleuchtmitteln zur Erzeugung von Weißlicht aktiv ist.

Das zweite Halbleiterleuchtmittel wird demnach im Fluoreszenzmodus zusammen mit dem ersten Halbleiterleuchtmittel aktiviert, wobei das zweite Halbleiterleuchtmittel dann Licht im zweiten Spektralbereich (Nebenspektralbereich, beispielsweise für die Blauzunge) zur optimalen Farbkontrastgebung im Fluoreszenzbild verwendet wird, während das zweite Halbleiterleuchtmittel im Weißlichtmodus in Kombination mit den weiteren Halbleiterleuchtmitteln zur Weißlichterzeugung aktiviert wird. Im Fall einer Fluoreszenzdiagnose mittels PPIX emittiert das zweite Halbleiterleuchtmittel zur Erzeugung der Blauzunge im blauen Spektralbereich.

Vorzugsweise weist die Vorrichtung des Weiteren eine Umschaltsteuerung zum Umschalten zwischen dem Fluoreszenzmodus und dem Weißlichtmodus auf, wobei die Umschaltsteuerung zum Umschalten zwischen dem Fluoreszenzmodus und dem Weißlichtmodus das zumindest eine erste Halbleiterleuchtmittel, das zumindest eine zweite Halbleiterleuchtmittel und das zumindest eine weitere Halbleiterleuchtmittel schaltet.

Während bei herkömmlichen Fluoreszenzdiagnosevorrichtungen zur Umschaltung zwischen dem Fluoreszenz- und dem Weißlichtmodus der Autoshutter des Bildaufnahmechips benutzt wird, hat die vorstehend genannte Maßnahme den Vorteil, dass auf einen Autoshutter verzichtet werden kann.

In einer weiteren bevorzugten Ausgestaltung sind in einem Speicher eine Mehrzahl von unterschiedlichen voreingestellten Verhältnissen aus der ersten und der zweiten Intensität abgespeichert, zwischen denen wahlweise umgeschaltet werden kann, wobei die Steuer- und Regeleinrichtung das jeweils gewählte voreingestellte Verhältnis konstant hält.

Wie bereits oben erwähnt, ist es in der Regel erforderlich, den optimalen Farbkontrast, d.h. die optimale Beimischung von Licht in einem Nebenspektralbereich der Anregung, beispielsweise von Blaulicht, in einer klinischen Evaluierung zu ermitteln. Nun kann der optimale Farbkontrast bei verschiedenen Anwendungen (z.B. verschiedenen Organen, verschiedenen diagnostischen Aufgaben, ...) unterschiedlich ausfallen, d.h. es kann unterschiedliche voreingestellte Intensitätsverhältnisse für unterschiedliche Organe geben. Dem trägt die vorstehend genannte Maßnahme vorteilhafterweise Rechnung. Die Steuer- und Regeleinrichtung hält auch in diesem Fall das jeweilige voreingestellte Intensitätsverhältnis konstant.

Ebenso bevorzugt ist es, wenn die Lichtquelle eine Mehrzahl von zweiten Halbleiterleuchtmitteln aufweist, die jeweils Licht in unterschiedlichen voreingestellten zweiten Spektralbereichen erzeugen, wobei zwischen den unterschiedlichen zweiten Spektralbereichen wahlweise umgeschaltet werden kann.

Diese Ausgestaltung hat zum einen den Vorteil, dass die Fluoreszenzdiagnosevorrichtung auf unterschiedliche Fluoreszenzfarbstoffe ausgelegt werden kann, weil sie Licht in unterschiedlichen Nebenspektralbereichen erzeugen kann. Aber auch im Fall ein und desselben Fluoreszenzfarbstoffes hat diese Maßnahme den Vorteil, dass dem Fluoreszenzbild Licht aus verschiedenen Nebenspektralbereichen beigemischt werden kann, um einen für die Diagnose optimalen Farbkontrast zu erhalten.

In einer weiteren bevorzugten Ausgestaltung ist zwischen dem zumindest einen ersten Halbleiterleuchtmittel und dem zumindest einen zweiten Halbleiterleuchtmittel ein Strahlkombinationselement angeordnet.

Hierbei ist von Vorteil, dass das erste Halbleiterleuchtmittel und das zumindest eine zweite Halbleiterleuchtmittel nicht auf einer gemeinsamen optischen Achse angeordnet werden müssen, was zu räumlichen Konflikten führen würde, sondern die beiden Halbleiterleuchtmittel können mit sich schneidenden Abstrahlachsen angeordnet werden. Das Strahlkombinationselement kann beispielsweise eine Planplatte mit reflektierender Vorderseite und für Licht im betreffenden Spektralbereich durchlässiger Rückseite sein.

Des Weiteren kann dem zumindest einen Halbleiterleuchtmittel und dem zumindest einen zweiten Halbleiterleuchtmittel zumindest ein optisches Element zur Kollimation nachgeordnet sein, das vorzugsweise asphärisiert ist.

Eine Fokussieroptik hat den Vorteil, dass das von den Halbleiterleuchtmitteln ungebündelt emittierte Licht zur Einkopplung in einen Lichtleiter gebündelt werden kann, der mit einem Endoskop, Exoskop oder einem Mikroskop verbunden ist, so dass Intensitätsverluste bei der Einkopplung des Lichts in den Lichtleiter so gering wie möglich gehalten werden können.

In einer weiteren bevorzugten Ausgestaltung ist der zumindest eine zweite Spektralbereich, in dem das zumindest eine zweite Halbleiterleuchtmittel Licht erzeugt, schmalbandig, und im Fall der Verwendung der Fluoreszenzdiagnosevorrichtung mit dem Fluoreszenzfarbstoff PPIX erzeugt das zweite Halbleiterleuchtmittel Licht vorzugsweise mit einer Peak-Wellenlänge im Bereich von etwa 400 nm bis etwa 500 nm, vorzugsweise von etwa 450 nm.

Die Schmalbandigkeit des zweiten Halbleiterleuchtmittels zur Erzeugung des Nebenspektralbereichs (im Falle von PPIX die Blauzunge) hat den Vorteil, dass bei geschicktem Abgleich zwischen den Intensitäten des Nebenspektralbereichs und des Hauptspektralbereichs eine Rotfluoreszenz mit einem anderen Farbkontrast im Fluoreszenzbild erscheint, wenn die Fluoreszenz intensiv ist, was auf malignes Gewebe hinweist, als wenn die Fluoreszenz schwach ist, d.h. kein spezifischer Befund vorliegt.

In einer weiteren bevorzugten Ausgestaltung kann das voreingestellte Verhältnis aus erster Intensität und zweiter Intensität während des Betriebes kontinuierlich oder diskret verändert werden.

Es wurde oben erwähnt, dass der Benutzer keine freie Wahl des Intensitätsverhältnisses haben sollte, da dies die Gefahr falsch positiver und vor allem falsch negativer Diagnosestellung birgt. Die vorstehende Maßnahme hat jedoch den Vorteil, beispielsweise bei der Schulung von Ärzten, dass in nicht-diagnostischen Situationen auch veränderte bzw. zusätzliche Werte vorgegeben werden können. Hierdurch kann bei einer Schulung demonstriert werden, wie sich eine Änderung des voreingestellten Verhältnisses der Intensitäten im Haupt- und Nebenspektralbereich auf den Farbkontrast und damit auf die Diagnose auswirken können. Auch für erfahrene Spezialisten auf dem Gebiet der Fluoreszenzdiagnose kann diese Wahlmöglichkeit nützlich sein.

Der Anwender kann einen anderen Wert für das Intensitätsverhältnis zwischen Haupt- und Nebenspektralbereich als das voreingestellte Intensitätsverhältnis wählen, wobei das gewählte Intensitätsverhältnis beispielsweise um einen Faktor zwischen 1,05 und 100, bevorzugt 1,1 und 20 gegenüber dem voreingestellten bzw. voreingestellten abweichen kann. Dabei kann des Weiteren vorgesehen sein, dass zwischen verschiedenen fest voreingestellten Intensitätsverhältnissen gewählt werden kann, dass andere als die fest voreingestellten Intensitätsverhältnisse gewählt werden können oder dass das voreingestellte Intensitätsverhältnis zeitlich befristet umgeschaltet werden kann.

Weitere Vorteile und Merkmale ergeben sich aus der nachfolgenden Beschreibung und der beigefügten Zeichnung.

Es versteht sich, dass die vorstehend genannten und nachstehend noch zu erläuternden Merkmale nicht nur in der jeweils angegebenen Kombination, sondern auch in anderen Kombinationen oder in Alleinstellung verwendbar sind, ohne den Rahmen der vorliegenden Erfindung zu verlassen.

Ausführungsbeispiele der Erfindung sind in der Zeichnung dargestellt und werden mit Bezug auf diese hiernach näher beschrieben. Es zeigen:
- Fig. 1: eine endoskopische Vorrichtung zur Fluoreszenzdiagnose in einem Blockschaltbild;
- Fig. 2: ein Ausführungsbeispiel einer Lichtquelle der Vorrichtung in Fig. 1;
- Fig. 3: ein weiteres Ausführungsbeispiel einer Lichtquelle der Vorrichtung in Fig. 1;
- Fig. 4: ein Diagramm zur Veranschaulichung der Auswirkung einer Beimischung von Licht in einem Nebenspektralbereich zum Hauptspektralbereich der Anregung in einem Fluoreszenzbild; und
- Fig. 5: ein Ablaufdiagramm einer Regelung, mit der ein voreingestelltes Intensitätsverhältnis von Licht im Hauptspektralbereich zu Licht im Nebenspektralbereich konstant gehalten wird.

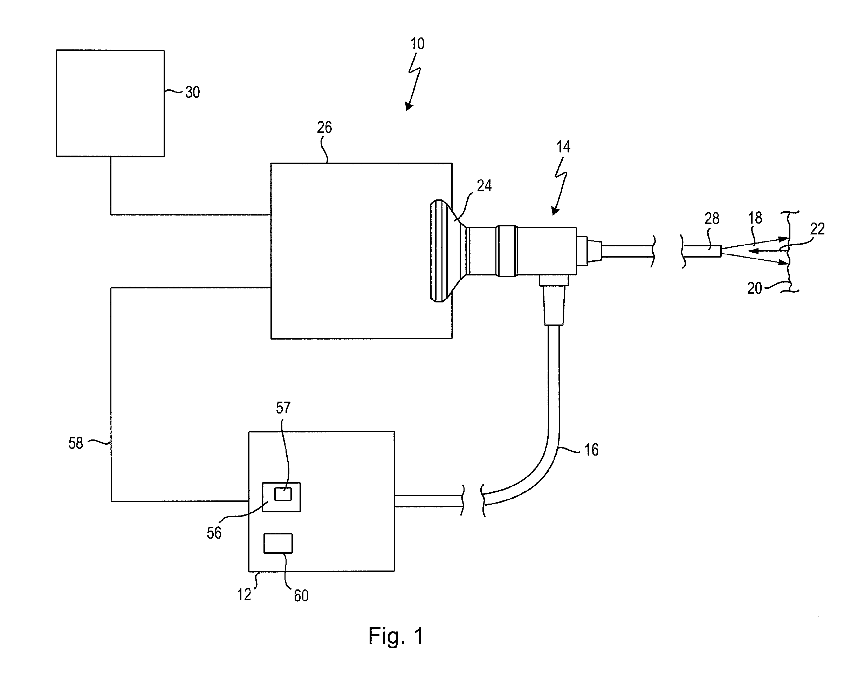

In Fig. 1 ist eine mit dem allgemeinen Bezugszeichen 10 versehene Vorrichtung zur Fluoreszenzdiagnose gezeigt. Im gezeigten Ausführungsbeispiel ist die Vorrichtung 10 eine endoskopische Fluoreszenzdiagnosevorrichtung.

Die Vorrichtung 10 weist eine Lichtquelle 12 auf, die in einem Fluoreszenzmodus Licht in einem ersten Spektralbereich und Licht in einem zweiten Spektralbereich emittiert, wie später noch beschrieben werden wird. Dabei ist der zweite Spektralbereich von dem ersten Spektralbereich zumindest teilweise getrennt.

Die Vorrichtung 10 weist weiterhin ein Endoskop 14 auf, das mit der Lichtquelle 12 über einen Lichtleiter in Form eines Lichtleitkabels 16 verbunden ist.

Von der Lichtquelle 12 emittiertes Licht wird über das Lichtleitkabel 16 in das Endoskop 14 eingespeist und von diesem, wie mit einem Lichtkegel 18 angedeutet, auf ein zu beobachtendes Areal 20, beispielsweise ein Gewebeareal im menschlichen oder tierischen Körper, gerichtet.

Das von der Lichtquelle 12 erzeugte Licht im ersten Spektralbereich dient zur Anregung einer Fluoreszenz eines im Areal 20 angereicherten Fluoreszenzfarbstoffes. Ein solcher Fluoreszenzfarbstoff ist beispielsweise PPIX, wie oben beschrieben wurde. Der vorstehend genannte erste Spektralbereich ist demnach der Spektralbereich der Fluoreszenzanregung des Fluoreszenzfarbstoffes. Dieser erste Spektralbereich wird nachfolgend auch Hauptspektralbereich genannt.

Durch das Licht im Hauptspektralbereich wird der im Areal 20 vorhandene Fluoreszenzfarbstoff zur Fluoreszenz angeregt. Das von dem Fluoreszenzfarbstoff emittierte Fluoreszenzlicht, das mit einem Pfeil 22 angedeutet ist, wird von dem Endoskop 14 empfangen und durch das (nicht gezeigte) optische System des Endoskops, das durch Linsen oder eine Faseroptik gebildet sein kannn, zu einem Okular 24 geführt, an dem eine Bilderfassungseinrichtung 26, vorzugsweise eine Kamera, angeschlossen ist.

Es versteht sich, dass die Bilderfassungseinrichtung 26 auch in das Endoskop 14 integriert sein kann, wobei heutzutage miniaturisierte Kameras erhältlich sind, die sogar in die Spitze 28 des Endoskops 14 integriert werden können.

Des Weiteren versteht es sich, dass die Vorrichtung 10 anstelle des Endoskops 14 ein Mikroskop oder ein Exoskop aufweisen kann.

Die Bilderfassungseinrichtung 26 ist mit einer Bildwiedergabeeinrichtung 30, beispielsweise einem Monitor, verbunden.

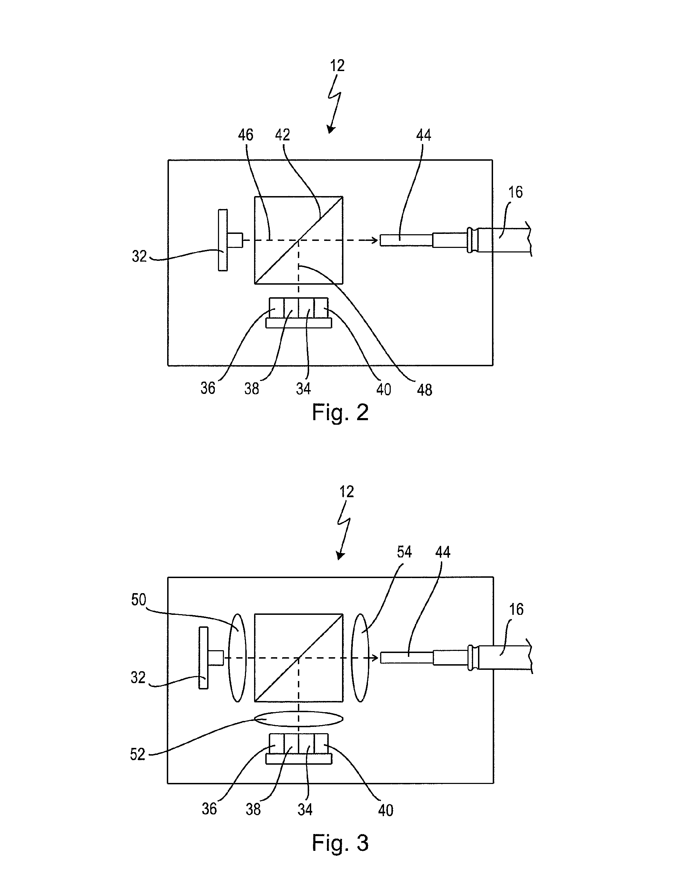

Bevor weitere Einzelheiten der Vorrichtung 10 beschrieben werden, werden zunächst mit Bezug auf Fig. 2 und 3 Ausführungsbeispiele der Lichtquelle 12 beschrieben.

Die Lichtquelle 12 weist ein erstes Halbleiterleuchtmittel 32 auf, das Licht in dem ersten Spektralbereich, d.h. dem Hauptspektralbereich der Fluoreszenzanregung emittiert.

In dem Beispiel des Fluoreszenzfarbstoffes PPIX emittiert das Halbleiterleuchtmittel 32 Licht im Ultravioletten in einem schmalbandigen Bereich mit einer Peak-Wellenlänge von etwa 405 nm.

Das erste Halbleiterleuchtmittel 32 kann als Leuchtdiode (anorganisch oder organisch), als Laserdiode, als Leuchtdiodenarray oder als Laserdiodenarray ausgebildet sein.

Die Lichtquelle 12 weist zumindest ein zweites Halbleiterleuchtmittel 34 auf, sowie weitere Halbleiterleuchtmittel 36, 38 und 40. Die Halbleiterleuchtmittel 34, 36, 38 und 40 bilden ein Array und sind jeweils als eine oder mehrere Leuchtdioden (organisch oder anorganisch) oder als Laserdioden ausgebildet.

Das zweite Halbleiterleuchtmittel 34 emittiert Licht in einem zweiten Spektralbereich, der von dem ersten Spektralbereich, in dem das erste Halbleiterleuchtmittel 32 emittiert, zumindest teilweise getrennt ist. Der zweite Spektralbereich wird nachfolgend auch als Nebenspektralbereich bezeichnet. Das zweite Halbleiterleuchtmittel 34 dient dazu, Licht im Nebenspektralbereich zu emittieren, das durch Streuung oder Reflexion vom Areal 20 remittiert und im Fluoreszenzmodus dem Fluoreszenzlicht beigemischt wird, um einen für die Diagnose optimalen Farbkontrast im Fluoreszenzbild zu erhalten.

Im vorliegenden Ausführungsbeispiel emittiert das zweite Halbleiterleuchtmittel 34 Licht im Blauen (sogenannte Blauzunge) in einem schmalbandigen Spektralbereich mit einer Peak-Wellenlänge von etwa 450 nm. Das von dem zweiten Halbleiterleuchtmittel 34 emittierte Licht ist somit etwas langwelliger als das von dem ersten Halbleiterleuchtmittel 32 emittierte Licht.

Bei den weiteren Halbleiterleuchtmitteln 36, 38 und 40 handelt es sich beispielsweise um einzelne Leuchtdioden (organisch oder anorganisch), wobei beispielsweise das Halbleiterleuchtmittel 36 eine im grünen Spektralbereich emittierende Leuchtdiode, das Halbleiterleuchtmittel 38 eine im roten Spektralbereich emittierende Leuchtdiode und das Halbleiterleuchtmittel 40 eine Weißlicht emittierende Leuchtdiode ist.

In einem Weißlichtmodus der Vorrichtung 10 erzeugen die Halbleiterleuchtmittel 34, 36, 38 und 40 Weißlicht.

Im Fluoreszenzmodus wird lediglich das zweite Halbleiterleuchtmittel 34 zusammen mit dem ersten Halbleiterleuchtmittel 32 aktiviert, und im Weißlichtmodus wird das zweite Halbleiterleuchtmittel 34 mit den weiteren Halbleiterleuchtmitteln 36, 38 und/oder 40 zur Erzeugung von Weißlicht aktiviert.

Die Lichtquelle 12 weist des Weiteren ein Strahlkombinationselement 42 auf, das auf seiner Seite, die dem ersten Halbleiterleuchtmittel 32 zugewandt ist, durchlässig und auf der den Halbleiterleuchtmitteln 34, 36, 38 und 40 zugewandten Seite reflektierend ist.

Das Strahlkombinationselement 42 kombiniert das von den Halbleiterleuchtmitteln 32 sowie 34, 36, 38 und 40 emittierte Licht zur gemeinsamen Einkopplung in ein Ende einer oder mehrerer Lichtfasern 44 des Lichtleitkabels 16 zur Weiterleitung zu dem Endoskop 14.

Aufgrund des Strahlkombinationselements 42 können die einzelnen Halbleiterleuchtmittel 32, 34, 36, 38 und 40 so zueinander angeordnet werden, dass ihre Abstrahlachsen 46 bzw. 48 winklig, insbesondere rechtwinklig, zueinander stehen.

Fig. 3 zeigt ein gegenüber dem Ausführungsbeispiel in Fig. 2 abgewandeltes Ausführungsbeispiel der Lichtquelle 12, das sich von dem Ausführungsbeispiel in Fig. 2 dadurch unterscheidet, dass dem ersten Halbleiterleuchtmittel 32 eine Optik 50, dem zweiten Halbleiterleuchtmittel 34 und den weiteren Halbleiterleuchtmitteln 36, 38 und 40 eine Optik 52 und dem Strahlkombinationselement 42 eine Optik 54 nachgeordnet sind. Die Optiken 50, 52 bzw. 54 können asphärisiert sein. Insbesondere wenn die Halbleiterleuchtmittel 32 bzw. 34, 36, 38 und 40 als Arrays von Leuchtdioden oder Laserdioden ausgebildet sind, haben die Optiken 50, 52 und 54 den Vorteil, dass sie das emittierte Licht auf einen schmalen Querschnitt kollimieren und das Licht damit verlustfrei in die Faser bzw. Fasern 44 des Lichtleitkabels 16 eingekoppelt werden kann.

Im Fluoreszenzmodus der Vorrichtung 10 ist es für einen für eine sichere Diagnose optimalen Farbkontrast wesentlich, dass das Verhältnis der Intensität des von dem ersten Halbleiterleuchtmittel 32 emittierten Fluoreszenzanregungslichts und der Intensität des von dem zweiten Halbleiterleuchtmittel 34 emittierten Lichts im Nebenspektralbereich einen bestimmten Wert hat oder zumindest in einem bestimmten schmalen Wertebereich liegt. Das optimale Intensitätsverhältnis zwischen dem Licht im ersten Spektralbereich (Hauptspektralbereich) und zweiten Spektralbereich (Nebenspektralbereich) wird optimalerweise in klinischen Studien ermittelt. Das so ermittelte optimale Intensitätsverhältnis (oder der so ermittelte optimale Bereich von Intensitätsverhältnissen) wird in einem Speicher, beispielsweise in der Lichtquelle 12, als voreingestelltes Intensitätsverhältnis abgespeichert.

Während des Betriebs der Lichtquelle 12 in einer Diagnosesitzung oder über die Lebensdauer der Lichtquelle 12, d.h. der Halbleiterleuchtmittel 32, 34, können sich die optischen Eigenschaften der Halbleiterleuchtmittel 32 und 34 ändern, insbesondere unterschiedlich zueinander ändern, was zur Folge hat, dass das vorstehend erwähnte Intensitätsverhältnis von Licht im Hauptspektralbereich und Licht im Nebenspektralbereich sich ebenfalls ändert. Eine derartige Änderung hat eine Veränderung des Farbkontrastes im Fluoreszenzbild zur Folge, was die Deutung des Fluoreszenzbildes bei der Diagnose auf malignes oder benignes Gewebe erschwert und sogar die Gefahr von Falsch-Diagnosen mit sich bringen kann.

Vor diesem Hintergrund weist die Vorrichtung 10 eine Steuer- oder Regeleinrichtung 56 auf, die das vorstehend genannte voreingestellte Intensitätsverhältnis des Lichts im Hauptspektralbereich und des Lichts im Nebenspektralbereich konstant hält.

Das voreingestellte Intensitätsverhältnis kann dabei dadurch konstant gehalten werden, dass die jeweilige Beaufschlagung des Halbleiterleuchtmittels 32 und des Halbleiterleuchtmittels 34 mit Strom oder Spannung gesteuert oder geregelt wird. Da bei Halbleiterleuchtmitteln auch die Temperatur einen Einfluss auf die emittierte Intensität hat, kann auch vorgesehen sein, dass die Steuer- oder Regeleinrichtung 56 die Temperatur der Halbleiterleuchtmittel 32, 34 im Zusammenwirken mit einer nicht gezeigten Kühlung/Heizung steuert oder regelt.

Die Steuer- oder Regeleinrichtung 56 ist in die Lichtquelle 12 integriert.

Die Steuer- oder Regeleinrichtung 56 kann als bloße Steuereinrichtung ausgebildet sein, bevorzugt ist sie jedoch als Regeleinrichtung ausgebildet.

Insbesondere im Falle der Ausgestaltung als Regeleinrichtung ist die Steuer- oder Regeleinrichtung 56 dazu ausgelegt, das voreingestellte Verhältnis aus der Intensität des Lichts im ersten Spektralbereich und der Intensität des Lichts im zweiten Spektralbereich auf Änderungen hin zu überwachen und bei detektierten Änderungen das aktuelle oder Ist-Verhältnis der Intensitäten auf das voreingestellte Verhältnis zurückzusetzen bzw. nachzuführen. Dies kann, wie später noch beschrieben wird, dadurch realisiert sein, dass die Steuer- oder Regeleinrichtung 56 eine Messeinrichtung 57 zum Messen der von dem Halbleiterleuchtmittel 32 emittierten Intensität und zum Messen der von dem Halbleiterleuchtmittel 34 emittierten Intensität aufweist.

Es kann aber auch die Bilderfassungseinrichtung 26, die das Fluoreszenzbild erfasst und deren Bildsignale der Steuer- oder Regeleinrichtung 56 über eine Verbindung 58 zugeführt werden, zur Überwachung des voreingestellten Intensitätsverhältnisses genutzt werden. Die Steuer- oder Regeleinrichtung 56 wertet die von der Bilderfassungseinrichtung 26 übermittelten Bildsignale in kurzen Zeitintervallen dann aus, um zu prüfen, ob sich das voreingestellte Intensitätsverhältnis ändert. Im Falle einer Änderung des voreingestellten Intensitätsverhältnisses steuert die Steuer- oder Regeleinrichtung 56 entsprechend die Halbleiterleuchtmittel 32 und/oder 34 unabhängig voneinander an, um durch eine angepasste Bestromung derselben das voreingestellte Intensitätsverhältnis wieder herzustellen.

Alternativ zu der vorstehend beschriebenen Ausgestaltung der Steuer-oder Regeleinrichtung als Regeleinrichtung kann sie auch als bloße Steuereinrichtung ausgebildet sein. In diesem Fall ist ein vorher ermittelter typischer zeitlicher Verlauf von Änderungen der von den Halbleiterleuchtmitteln 32, 34 emittierten Intensitäten gespeichert, beispielsweise in einem Speicherbaustein der Steuer- oder Regeleinrichtung 56. Ein solcher typischer zeitlicher Verlauf von Änderungen der emittierten Intensitäten kann beispielsweise dadurch ermittelt werden, dass der zeitliche Verlauf der emittierten Intensitäten des ersten und des zweiten Halbleiterleuchtmittels 32 bzw. 34 vorab ermittelt wird. Der beispielsweise in der Steuer- oder Regeleinrichtung 56 abgespeicherte typische zeitliche Verlauf wird dann zur Berechnung von Korrekturwerten herangezogen, mit denen die Steuer- oder Regeleinrichtung 56 das erste Halbleiterleuchtmittel 32 und/oder das zweite Halbleiterleuchtmittel 34 entsprechend ansteuert, um das voreingestellte Intensitätsverhältnis konstant zu halten.

Das Umschalten zwischen dem Fluoreszenzmodus und dem Weißlichtmodus erfolgt bei der Vorrichtung 10 durch das entsprechende Schalten des zumindest einen ersten Halbleiterleuchtmittels 32, des zumindest einen zweiten Halbleiterleuchtmittels 34 und der weiteren Halbleiterleuchtmittel 36, 38 und 40. Hierzu ist eine Umschaltsteuerung 60 vorhanden, die in die Lichtquelle 12 integriert sein kann.

In einem Speicher, beispielsweise in einem in der Steuer- oder Regeleinrichtung 56 vorhandenen Speicherbaustein, können eine Mehrzahl von unterschiedlichen voreingestellten Verhältnissen aus der ersten Intensität im Hauptspektralbereich und der zweiten Intensität im Nebenspektralbereich abgespeichert sein, zwischen denen der Benutzer wahlweise umschalten kann, wobei die Steuer- oder Regeleinrichtung 56 das jeweils gewählte voreingestellte Verhältnis dann konstant hält.

In Abwandlung des Ausführungsbeispiels der Lichtquellen 12 in Fig. 2 und 3 kann die Lichtquelle 12 nicht nur ein zweites Halbleiterleuchtmittel 34 aufweisen, sondern eine Mehrzahl von zweiten Halbleiterleuchtmitteln 34, wozu auch die Halbleiterleuchtmittel 36, 38 oder 40 verwendet werden können, die jeweils Licht in unterschiedlichen vorbestimmten zweiten Spektralbereichen, d.h. Nebenspektralbereichen, Licht erzeugen, so dass jeweils Licht einer oder mehrerer dieser zweiten Halbleiterleuchtmittel wahlweise eingeschaltet werden können, um dem Fluoreszenzlicht Licht in einem unterschiedlichen Nebenspektralbereich beizumischen, wobei die Erfindung vorsieht, dass das Intensitätsverhältnis jedoch konstant bleiben muss.

Schließlich kann es auch vorgesehen sein, dass das voreingestellte Intensitätsverhältnis zwischen Hauptspektralbereich und Nebenspektralbereich während des Betriebes der Vorrichtung 10 kontinuierlich oder diskret verändert werden kann, wobei dann das veränderte Intensitätsverhältnis von der Steuer- oder Regeleinrichtung 56 konstant gehalten wird. Die wahlweise Änderung des voreingestellten Verhältnisses aus den Intensitäten im Hauptspektralbereich und Nebenspektralbereich kann insbesondere zeitlich begrenzt sein, während nach einer vorbestimmten Zeit wieder auf das voreingestellte Intensitätsverhältnis umgeschaltet wird.

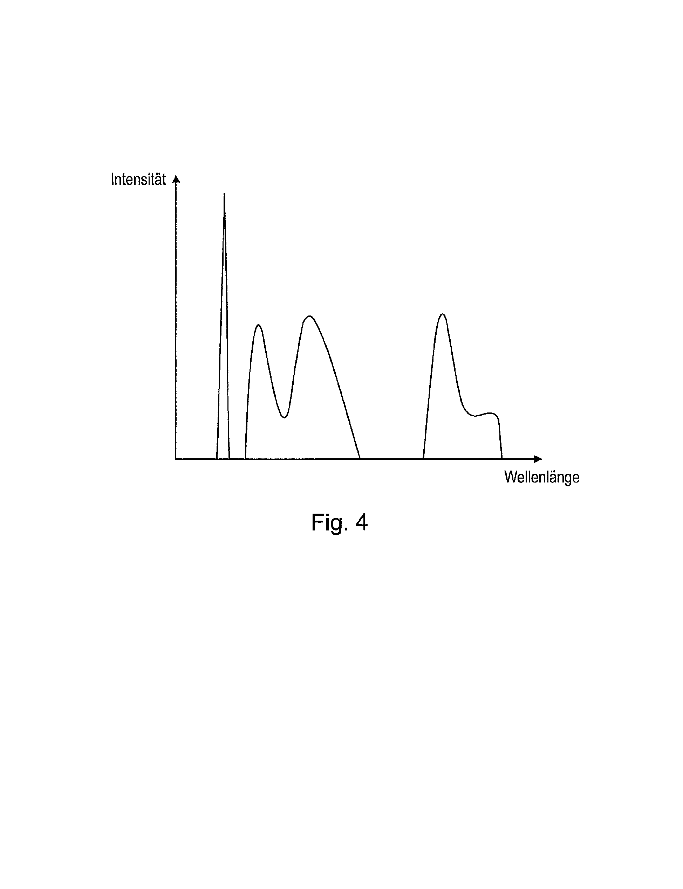

Fig. 4 zeigt schematisch ein Diagramm, wie sich der Nebenspektralbereich (mittlere Kurve) auf das Fluoreszenzbild auswirkt. Die Schmalbandigkeit der Emission des zweiten Halbleiterleuchtmittels 34 zur Erzeugung des Nebenspektralbereichs (im Falle von PPIX die Blauzunge) hat den Vorteil, dass bei geschicktem Abgleich zwischen den Intensitäten des Nebenspektralbereichs und des Hauptspektralbereichs eine Rotfluoreszenz mit anderem Farbkontrast im Fluoreszenzbild erscheint, wenn die Fluoreszenz intensiv ist, was auf malignes Gewebe hinweist, als wenn die Fluoreszenz schwach ist, d.h. kein spezifischer Befund vorliegt.

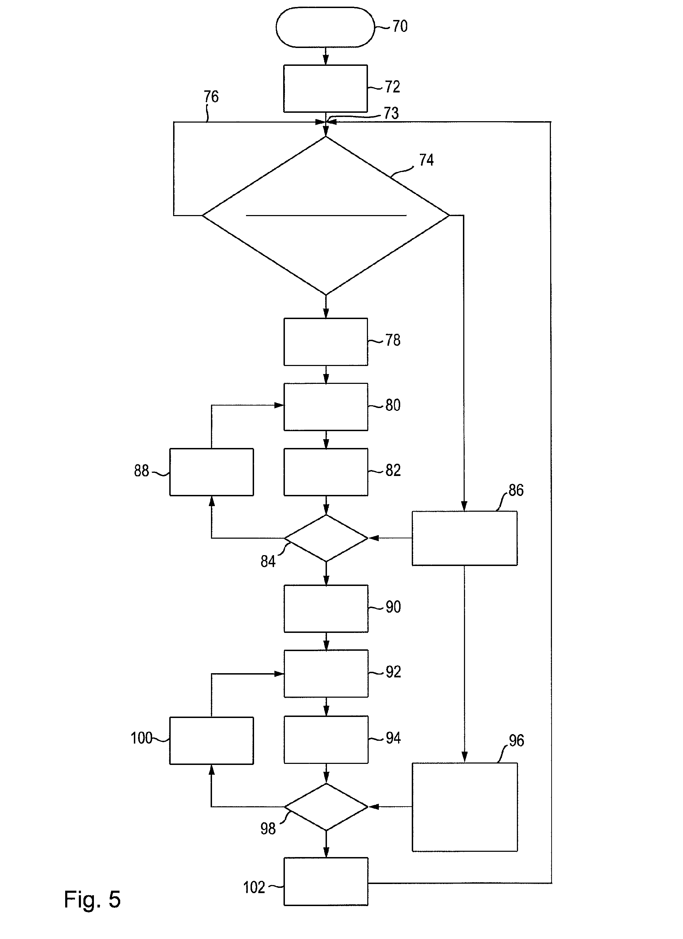

In Fig. 5 ist ein Ablaufdiagramm gezeigt, wie ein voreingestelltes Intensitätsverhältnis aus der Intensität im Hauptspektralbereich und der Intensität im Nebenspektralbereich konstant gehalten werden kann. Der Anwender hat dabei keinen Einfluss auf den Ablauf der nachfolgend beschriebenen Regelung. Die Regelung einschließlich Überwachung des voreingestellten Intensitätsverhältnisses läuft dabei in einem sehr kurzen Zeitintervall (beispielsweise Millisekunden) ab, so dass die Regelung den Anwender während des Gebrauchs der Vorrichtung 10 nicht beeinträchtigt.

Durch Einschalten 70 der Lichtquelle 12 werden im Fluoreszenzmodus das erste Halbleiterleuchtmittel 32 und das zweite Halbleiterleuchtmittel 34 eingeschaltet (Bezugszeichen 72). Nach dem Einschalten der Halbleiterleuchtmittel 32 und 34 ist der Startpunkt 73 der Regelung erreicht. Die Halbleiterleuchtmittel 32 und 34 emittieren Licht im Hauptspektralbereich und Nebenspektralbereich mit einem aktuellen oder IstIntensitätsverhältnis. Bei 74 wird nun geprüft, ob das aktuelle oder Ist-Intensitätsverhältnis von dem voreingestellten Intensitätsverhältnis abweicht. Ist dies nicht der Fall, wird über 76 wieder zum Startpunkt zurückgekehrt.

Die Überprüfung des aktuellen oder Ist-Intensitätsverhältnisses bei 74 wird zu Beginn, d.h. nach dem Einschalten der Lichtquelle 70, und nach beliebigen Zeitintervallen sowie bei manueller Änderung der Helligkeit der Lichtquelle 12 durchgeführt.

Ergibt die Überprüfung bei 74 eine Abweichung oder Änderung des aktuellen oder Ist-Verhältnisses vom voreingestellten Intensitätsverhältnis, findet folgender weiterer Ablauf der Regelung durch die Steuer- oder Regeleinrichtung 56 statt.

Bei 78 wird zunächst das zweite Halbleiterleuchtmittel 34, das Licht im Nebenspektralbereich emittiert, ausgeschaltet, so dass bei 80 nur noch das erste Halbleiterleuchtmittel 32, das Licht im Hauptspektralbereich emittiert, eingeschaltet ist. Bei 82 wird die Intensität des von dem ersten Halbleiterleuchtmittel 32 emittierten Lichtes mittels der Messeinrichtung 57 gemessen. Die Messung kann beispielsweise über einen optischen Sensor, beispielsweise eine Photodiode der Messeinrichtung 57, erfolgen. Der gemessene Wert wird anschließend mit für das voreingestellte Intensitätsverhältnis geltenden Referenzwerten (Bezugszeichen 86) bei 84 verglichen. Treten Abweichungen der gemessenen Intensität zu den hinterlegten Referenzwerten auf, wird bei 88 die Bestromung des ersten Halbleiterleuchtmittels 32 entsprechend angepasst.

Ergibt der Vergleich bei 84, dass die von dem ersten Halbleiterleuchtmittel 32 emittierte Intensität korrekt ist, wird bei 90 das erste Halbleiterleuchtmittel 32 ausgeschaltet, während nun bei 92 nur das zweite Halbleiterleuchtmittel 34 eingeschaltet ist. Im folgenden Verlauf des Ablaufdiagramms wird nun der geeignete Intensitätswert der Emission des zweiten Halbleiterleuchtmittels 34 ermittelt. Dieser Vorgang erfolgt analog zur Ermittlung des korrekten Intensitätswertes des ersten Halbleiterleuchtmittels 32, d.h. bei 94 wird die Intensität der Emission des zweiten Halbleiterleuchtmittels 34 gemessen, mit Referenzwerten (Bezugszeichen 96) für das voreingestellte Intensitätsverhältnis und ggf. einem zusätzlich einstellbaren Intensitäts-Offset für das zweite Halbleiterleuchtmittel 34 bei 98 verglichen und, falls erforderlich, wird bei 100 die Bestromung des zweiten Halbleiterleuchtmittels 34 entsprechend geändert. Ergibt der Vergleich bei 98, dass die von dem zweiten Halbleiterleuchtmittel 34 emittierte Intensität korrekt ist, wird bei 102 das erste Halbleiterleuchtmittel 32 zugeschaltet, und die Regelung springt wieder an den Startpunkt 73 zurück, und die Überwachung und Regelung des voreingestellten Intensitätsverhältnisses beginnt von neuem.

## Patentansprüche

1. Endoskopische, exoskopische oder mikroskopische Vorrichtung zur Fluoreszenzdiagnose, mit einer Lichtquelle (12), die dazu ausgelegt ist, in einem Fluoreszenzmodus Licht in einem ersten Spektralbereich und Licht in einem zweiten Spektralbereich zu emittieren, wobei der zweite Spektralbereich von dem ersten Spektralbereich zumindest teilweise getrennt ist, wobei die Lichtquelle (12) zumindest ein erstes Halbleiterleuchtmittel (32) aufweist, das im Fluoreszenzmodus das Licht in dem ersten Spektralbereich emittiert, **dadurch gekennzeichnet, dass** die Lichtquelle (12) zumindest ein zweites Halbleiterleuchtmittel (34) aufweist, das in dem Fluoreszenzmodus das Licht in dem zweiten Spektralbereich emittiert, und mit einer Steuer- oder Regeleinrichtung (56), die ein voreingestelltes Verhältnis aus einer ersten Intensität des Lichts im ersten Spektralbereich und einer zweiten Intensität des Lichtes im zweiten Spektralbereich konstant hält.

2. Vorrichtung nach Anspruch 1, **dadurch gekennzeichnet, dass** die Steuer- oder Regeleinrichtung (56) dazu ausgelegt ist, das voreingestellte Verhältnis aus der ersten und der zweiten Intensität auf Änderungen hin zu überwachen und bei detektierten Änderungen das Ist-Verhältnis auf das voreingestellte Verhältnis zurückzusetzen.

3. Vorrichtung nach Anspruch 2, **dadurch gekennzeichnet, dass** die Steuer- oder Regeleinrichtung (56) eine Messeinrichtung (57) zum Messen der Intensitäten des von dem ersten Halbleiterleuchtmittel (32) und dem zumindest einen zweiten Halbleiterleuchtmittel (34) emittierten Lichts aufweist.

4. Vorrichtung nach Anspruch 2, weiterhin mit einer Bilderfassungseinrichtung (26) zur Erfassung eines Fluoreszenzbildes, **dadurch gekennzeichnet, dass** die Steueroder Regeleinrichtung (56) Bildsignale der Bilderfassungseinrichtung (26) auswertet, um das voreingestellte Verhältnis auf Änderungen zu überwachen.

5. Vorrichtung nach Anspruch 1, **dadurch gekennzeichnet, dass** in der Steuer- oder Regeleinrichtung (56) ein typischer zeitlicher Verlauf von Änderungen der Intensitäten des von dem ersten Halbleiterleuchtmittel (32) und dem zumindest einen zweiten Halbleiterleuchtmittel (34) emittierten Lichts gespeichert ist, und dass die Steuer- oder Regeleinrichtung (56) anhand des typischen zeitlichen Verlaufes der Änderungen Korrekturwerte berechnet, um das voreingestellte Verhältnis konstant zu halten.

6. Vorrichtung nach einem der Ansprüche 1 bis 5, **dadurch gekennzeichnet, dass** die Steuer- oder Regeleinrichtung (56) in die Lichtquelle (12) integriert ist.

7. Vorrichtung nach einem der Ansprüche 1 bis 6, **dadurch gekennzeichnet, dass** das zumindest eine erste Halbleiterleuchtmittel (32) eine Leuchtdiode, eine Laserdiode, ein Leuchtdiodenarray oder ein Laserdiodenarray ist.

8. Vorrichtung nach einem der Ansprüche 1 bis 7, **dadurch gekennzeichnet, dass** das zumindest eine zweite Halbleiterleuchtmittel (34) eine Leuchtdiode, eine Laserdiode, ein Leuchtdiodenarray oder ein Laserdiodenarray ist.

9. Vorrichtung nach einem der Ansprüche 1 bis 8, **dadurch gekennzeichnet, dass** die Lichtquelle zumindest ein, vorzugsweise mehrere weitere Halbleiterleuchtmittel (36, 38, 40) aufweist, die in einem Weißlichtmodus Weißlicht erzeugen.

10. Vorrichtung nach Anspruch 8 und 9, **dadurch gekennzeichnet, dass** das zweite Halbleiterleuchtmittel (34) eine einzelne Leuchtdiode ist, die im Fluoreszenzmodus nur mit dem ersten Halbleiterleuchtmittel (32) aktiv ist, und dass das zweite Halbleiterleuchtmittel (34) im Weißlichtmodus mit den weiteren Halbleiterleuchtmitteln (36, 38, 40) zur Erzeugung von Weißlicht aktiv ist.

11. Vorrichtung nach Anspruch 9 oder 10, **gekennzeichnet durch** eine Umschaltsteuerung (60) zum Umschalten zwischen dem Fluoreszenzmodus und dem Weißlichtmodus, wobei die Umschaltsteuerung (60) zum Umschalten zwischen dem Fluoreszenzmodus und dem Weißlichtmodus das zumindest eine erste Halbleiterleuchtmittel (32), das zumindest eine zweite Halbleiterleuchtmittel (34) und das zumindest eine weitere Halbleiterleuchtmittel (36, 38, 40) schaltet.

12. Vorrichtung nach einem der Ansprüche 1 bis 11, **dadurch gekennzeichnet, dass** eine Mehrzahl von unterschiedlichen voreingestellten Verhältnissen aus der ersten und der zweiten Intensität in einem Speicher abgespeichert sind, zwischen denen wahlweise umgeschaltet werden kann, und dass die Steuer- oder Regeleinrichtung (56) das jeweils gewählte voreingestellte Verhältnis konstant hält.

13. Vorrichtung nach einem der Ansprüche 1 bis 12, **dadurch gekennzeichnet, dass** die Lichtquelle (12) eine Mehrzahl von zweiten Halbleiterleuchtmitteln (34) aufweist, die jeweils Licht in unterschiedlichen voreingestellten zweiten Spektralbereichen erzeugen, wobei zwischen den unterschiedlichen zweiten Spektralbereichen wahlweise umgeschaltet werden kann.

14. Vorrichtung nach einem der Ansprüche 1 bis 13, **dadurch gekennzeichnet, dass** zwischen dem zumindest einen ersten Halbleiterleuchtmittel (32) und dem zumindest einen zweiten Halbleiterleuchtmittel (34) ein Strahlkombinationselement (42) angeordnet ist.

15. Vorrichtung nach einem der Ansprüche 1 bis 14, **dadurch gekennzeichnet, dass** dem zumindest einen ersten Halbleiterleuchtmittel (32) und dem zumindest einen zweiten Halbleiterleuchtmittel (34) zumindest ein optisches Element (50, 52) zur Kollimation nachgeordnet ist, das vorzugsweise asphärisiert ist.

16. Vorrichtung nach einem der Ansprüche 1 bis 15, **dadurch gekennzeichnet, dass** der zumindest eine zweite Spektralbereich, in dem das zumindest eine zweite Halbleiterleuchtmittel (34) Licht erzeugt, schmalbandig ist, vorzugsweise mit einer Peak-Wellenlänge im Bereich von etwa 400 nm bis etwa 500 nm, vorzugsweise von etwa 450 nm.

17. Vorrichtung nach einem der Ansprüche 1 bis 16, **dadurch gekennzeichnet, dass** das voreingestellte Verhältnis aus erster Intensität und zweiter Intensität während des Betriebes kontinuierlich oder diskret verändert werden kann.
